Europäisches Patentamt

European Patent Office

Office européen des brevets

(11) Veröffentlichungsnummer: **0 230 223 B1**

(12) **EUROPÄISCHE PATENTSCHRIFT**

(45) Veröffentlichungstag der Patentschrift: **23.09.92**

(21) Anmeldenummer: **87100068.3**

(22) Anmeldetag: **06.01.87**

(51) Int. Cl.⁵: **C12M 3/00**

(54) **Perifusionsgerät für die Züchtung lebender Zellen und Verfahren zur Perifusions-Kultivierung.**

(30) Priorität: **10.01.86 DE 3600487**

(43) Veröffentlichungstag der Anmeldung:
**29.07.87 Patentblatt 87/31**

(45) Bekanntmachung des Hinweises auf die
Patenterteilung:
**23.09.92 Patentblatt 92/39**

(84) Benannte Vertragsstaaten:
**AT BE CH DE FR GB IT LI LU NL SE**

(56) Entgegenhaltungen:
**FR-A- 2 393 849**

**BIOTECHNOLOGY AND BIOENGINEERING,
BAnd 23, 1981, Seiten 2703-2716, John Wiley
& Sons, Inc., New York, US; M.D. JENSEN:
"Production of anchorage-dependent cells.
Problems and their possible solutions"**

**ANALYTICAL BIOCHEMISTRY, Band 112,
1981, Seiten 117-127, Academic Press, Inc.,
New York, US; R.H. McMENAMY et al.:
"Perifusion system for isolated cells"**

(73) Patentinhaber: **Gebhardt, Rolf, Dr.
Dornäckerweg 13
W-7400 Tübingen 7(DE)**

(72) Erfinder: **Gebhardt, Rolf, Dr.
Dornäckerweg 13
W-7400 Tübingen 7(DE)**

(74) Vertreter: **Patentanwälte RUFF, BEIER und
SCHÖNDORF
Neckarstrasse 50
W-7000 Stuttgart 1(DE)**

**Beschreibung**

Die Erfindung betrifft ein Perifusionsgerät für die Züchtung bzw. Kultivierung lebender Zellen mit mindestens einer Kulturkammereinheit zur Aufnahme von flüssigem Kulturmedium und Gas, mindestens einem Oxygenator für das Kulturmedium, mindestens einer Flüssigkeitspumpe für das Kulturmedium und mindestens einem Reservoir für das Kulturmedium, sowie ein Verfahren zur Perifusions-Kultivierung.

Ein Perifusionsgerät mit drei verschiedenen Kammern ist aus FR-A-23 93 849 bekannt. In einem Zellkulturraum befindet sich eine Zellsuspension in einem ruhenden Kulturmedium. Auf der einen Seite dieser Zellkulturkammer grenzt ein von Kulturmedium durchströmter Kulturmediumraum an, wobei über eine semipermeable Membran ein Stoffaustausch möglich ist. Auf der anderen Seite der Zellkulturkammer befindet sich ein Gasraum, wobei ebenfalls Stoffaustausch durch eine gasdurchlässige aber wasserundurchlässige Flachmembran stattfindet. Da sowohl die Gasversorgung als auch der Transfer von Nährstoffen und der zu gewinnenden Hormone ausschließlich über die Membranen stattfindet, ist dieses Perifusionsgerät in seiner Leistungsfähigkeit stark begrenzt.

In der Dissertation des Anmelders "Hormonale Wechselwirkungen bei der Kontrolle hepatischer Enzymspiegel/ Primärkulturen von Hepatozyten als Modellsysteme für Untersuchungen zur Stoffwechselregulation", Fakultät für Chemie und Pharmazie der Eberhard-Karls-Universität zu Tübingen, 1980, Seiten 43 - 48, sowie in der Veröffentlichung von Rolf Gebhardt und Dieter Mecke "Perifused Monolayer Cultures of Rat Hepatocytes as an improved in Vitro System for Studies on Ureogenesis" in Experimental Cell Research 124 (1979) 349-359 ist ein Perifusionsgerät beschrieben. Dort besteht die Kulturkammereinheit aus einer flachen rechteckigen Schale mit Stopfen an zwei gegenüberliegenden Stirnseiten, durch die Einlaßnadeln zum Hindurchführen des Kulturmediums bzw. Gases geführt sind. Der Kammerboden ist mit mehreren Objektträgern für die Kultivierung der Zellen ausgelegt. Die Kulturkammer ist mit Deckel und Dichtung verschließbar. Sie ist über Schlauchleitungen mit dem Reservoir, einer Rollenpumpe und einem Oxygenator verbunden. Das bekannte Perifusionsgerät eignet sich für wissenschaftliche Versuche in kleinem Maßstab, hat jedoch gewisse Nachteile, die in den Veröffentlichungen bereits angesprochen sind.

Demgegenüber liegt der Erfindung die Aufgabe zugrunde, ein Perifusionsgerät zu schaffen, das die Kultivierung von Zellen in größerem Maßstab unter vergleichbaren Bedingungen ermöglicht.

Diese Aufgabe wird durch ein Perifusionsgerät der eingangs genannten Art gelöst, das dadurch gekennzeichnet ist, daß die Kulturkammereinheit in einen Flüssigkeitsraum und einen Gasraum unterteilt ist und hierzu der Raum für das Kulturmedium in der Kulturkammereinheit durch mindestens eine gaspermeable Wand von dem Raum für das Gas abgetrennt ist, in der Kulturkammereinheit im Raum für das Kulturmedium mindestens ein plattenförmiger Träger für eine Monolayer-Kultur im wesentlichen parallel zur gaspermeablen Wand angeordnet ist, die Kulturkammereinheit und die gaspermeable Wand im wesentlichen vertikal angeordnet sind, der Raum für das Kulturmedium für eine vollständige Füllung mit dem Kulturmedium im Betrieb ausgebildet ist, der Flüssigkeitsraum der Kulturkammereinheit laminar und im wesentlichen gleichmäßig über den gesamten Querschnitt des Raumes zwischen der gaspermeablen Wand und dem Träger mit Kulturmedium durchströmbar ist und an der Eintrittsstelle für das Kulturmedium im unteren Bereich der Kulturkammereinheit ein Gasabscheider vorgesehen ist.

Durch die Trennung der Kulturkammereinheit in einen Gasraum und einen Flüssigkeitsraum mit Hilfe einer gaspermeablen Wand wird die Handhabung des Perifusionsgerätes wesentlich erleichtert, da der Flüssigkeitskreislauf und der Gaskreislauf getrennt sind. Auch bei einer Bewegung der Kulturkammereinheit bleibt der Zellverband stets mit Kulturmedium bedeckt.

Im Gegensatz zum Perifusionsgerät nach der FR-A 23 93 849 ist es bei der Erfindung vorgesehen, nur zwei Räume bzw. Kammern pro Kulturkammereinheit vorzusehen und zu diesem Zweck in dem Raum, der mit Kulturmedium durchspült wird, auch die Zellkultur vorzusehen. Die Versorgung der Zellen mit Nährstoffen und Gas wird dadurch erheblich erleichtert, die Zellen kommen unmittelbar in Kontakt mit dem die Nährstoffe führenden Kulturmedium. Das Kulturmedium kann zudem vor der Einführung in die Kammer mit Gas beladen werden, so daß auch die Versorgung der Zellen mit Gas erheblich verbessert ist. Dadurch, daß sich parallel zur Flüssigkeitskammer eine Gaskammer befindet und zwischen beiden eine semipermeable Wand angeordnet ist, wird einer Verarmung des Kulturmediums an Gas während des Durchströmens der Kulturkammer entgegengewirkt, so daß der Verbrauch an Gas laufend kompensiert werden kann.

Die Kulturkammereinheit kann mindestens während der Befüllung mit Kulturmedium so angeordnet sein, daß der Einlaß für das Kulturmedium unten und der Auslaß oben an der Kulturkammereinheit sind, wodurch eine vollständige Befüllung gewährleistet ist. Durch die im wesentlichen vertikale Anordnung der Kulturkammereinheit und der gaspermeablen Wand ist eine raumsparende Aufstellung des Perifusionsgerätes möglich. Der im unteren Bereich der Kulturkammereinheit vorgesehene Gasabscheider verhindert das Eindringen von Gasblasen mit dem Kulturmedium in die Kulturkammer. Dies kann dadurch erreicht werden, daß im unteren Bereich der Kulturkammereinheit ein Zuleitungskanal für das Kulturmedium vorgesehen ist und Eintrittsstellen für das Kulturmedium vom Kanal in die Kammer unterhalb der Oberseite des Kanals angeordnet sind, so daß die Gasblasen an der oberen Wandung des Kanals aufgefangen und gegebenenfalls abgeleitet werden. Für die Zuleitung des Kulturmediums in die Kulturkammereinheit ist vorzugsweise eine Vielzahl von auf den gesamten Kammerquerschnitt verteilten Eintrittsöffnungen vorgesehen, wodurch eine gleichmäßige und laminare Strömung erreichbar ist. So kann eine Zuleitung für das Kulturmedium mit Vorteil entlang einer unteren Schmalseite geführt sein und mit der Kammer über eine Vielzahl von geschlitzten oder gelochten Öffnungen verbunden sein, wobei die Zuleitung vorzugsweise leicht schräg von unten nach oben verläuft.

Die Kulturkammereinheit ist vorzugsweise platten- bis tafelförmig ausgebildet, wobei die gaspermeable Trennwand im wesentlichen parallel zu einer großen Flächenseite der Kulturkammereinheit verläuft. Die gaspermeable Trennwand ist mit Vorteil bakteriendicht, so daß die Gefahr der Übertragung von Bakterien aus der Gasphase in die Flüssigphase nicht besteht. Vorzugsweise ist die gaspermeable Wand im wesentlichen unbeweglich ausgebildet. Hierzu kann die gaspermeable Wand an sich als flexible Membran ausgebildet sein, die mit einer Stützstruktur versteift ist. Dadurch kann erreicht werden, daß der für das Kulturmedium vorgesehene Raum in der Kulturkammereinheit im Volumen unveränderlich ist.

Es wurde gefunden, daß die Handhabbarkeit von Perifusionsgeräten weiterhin dadurch wesentlich verbessert werden kann, wenn mehrere, vorzugsweise mindestens fünf, Kulturkammern jeweils in getrennten Moduleinheiten vorgesehen sind, die von anderen Moduleinheiten und gegebenenfalls vom Vorratsbehälter für das Kulturmedium abnehmbar und insbesondere über lösbare Steckverbindungen mit diesen verbindbar sind. Dabei sind die Steckverbindungen vorzugsweise als Steckventile ausgebildet. Auf diese Weise ist es nicht mehr erforderlich, jede Kulturkammereinheit mit getrennten Zuleitungen zu versehen. Vielmehr können die Moduleinheiten unmittelbar miteinander verbunden werden, so daß insbesondere für eine Begasung, eine Thermisierung, einen Antrieb und/oder die Zuleitung von Kulturmedium ein Versorger bzw. Anschluß ausreicht. Eine Moduleinheit weist mindestens eine Kulturkammereinheit auf, jedoch vorzugsweise auch einen eigenen Oxygenator und insbesondere eine eigene Umwälzpumpe. Dies ist besonders günstig für die Schaffung vergleichbarer Verhältnisse in den Kulturkammern der einzelnen Moduleinheiten. Bei dieser Ausbildung der Kulturkammereinheiten in Form von Modulen sind die Kulturkammern ebenfalls mit besonderem Vorteil wiederum in einen Gasraum und einen Flüssigkeitsraum mittels einer gaspermeablen Wand getrennt. Die Moduleinheiten sind vorzugsweise flächig bis Plattenartig ausgebildet, so daß sie dicht nebeneinander angeordnet werden können, insbesondere mit gegenseitiger Berührung thermisch miteinander koppelbar sind. Auch dies wirkt sich vorteilhaft auf reproduzierbare und vergleichbare Ergebnisse aus. Die Moduleinheiten können jeweils mit einer Thermisiereinrichtung versehen sein, die sich vorzugsweise asymmetrisch und flächig auf einer Modulaußenseite befindet, insbesondere an der Seite, die dem Raum für das Gas benachbart ist. Durch Aneinanderlegen der Moduleinheiten kann erreicht werden, daß eine Thermisiereinrichtung auch die nicht mit einer Thermisiereinrichtung versehene Seite der benachbarten Moduleinheit durch den thermischen Kontakt mitthermisiert. Die Thermisierung kann elektrisch vorgenommen werden. Die Thermisiereinrichtungen weisen mit Vorteil jedoch Kanäle für die Durchleitung von Thermisierflüssigkeiten auf. Die letzte Moduleinheit einer Reihe kann dann an der freien Seite mit einer zusätzlichen Thermisiereinrichtung versehen sein, so daß ein symmetrischer Aufbau des gesamten Perifusionsgerätes in Bezug auf die Thermisierung erreicht wird.

Bei der bevorzugten Ausführungsform sind die im Raum für das Kulturmedium vorgesehenen Träger, die vorzugsweise als Trägerplatten ausgebildet sind, herausnehmbar. Sie können als Ganzes herausnehmbar sein oder in einzelne Teile unterteilt sein, die einzeln herausnehmbar sind. Zum Herausnehmen weist die Wandung der Kulturkammer vorzugsweise an einer Schmalseite eine verschließbare Öffnung auf, durch die der Träger herausnehmbar ist. Die Schmalseite ist vorzugsweise die Oberseite in der Betriebslage. Es können unterschiedliche Träger verwendet werden, die z.B. nach Art des Materials und der Oberflächenbeschaffenheit an wechselnde Anforderungen der jeweiligen Zellen insbesondere hinsichtlich der extrazellulären Matrix angepaßt sind und die Kultivierungsbedingungen günstig beeinflussen. Das Anheften bzw. Anwachsenlassen der Zellen an die Trägerplatte kann außerhalb der Kulturkammer vorgenommen werden oder auch innerhalb der Kammer in horizontaler Lage bei ruhendem oder nur sehr langsam fließendem Medium.

Dadurch, daß die Kammer in einen Gasraum und einen Flüssigkeitsraum unterteilt ist, kann die aus dem Kulturmedium bestehende Flüssigkeitsschicht über dem Zellverband bzw. der Trägerplatte sehr gering gehalten werden. In der Regel reicht ein für das Kulturmedium und den Zellverband zur Verfügung stehender Abstand zwischen Träger, insbesondere Trägerplatte, und gaspermeablen Wand in der Größe von ca. 0,8 - 1,2 mm, vorzugsweise ca. 1 mm aus. Dadurch kann der Raum für das Kulturmedium in der Kulturkammereinheit trotz großer Flächenausdehnung des Trägers für den Zellverband relativ klein gehalten werden. Bei einer Trägerplatte mit den Flächenmaßen von etwa 12 x 12 cm und entsprechend dimensionierter Kammer, reicht beispielsweise ein für das Kammermedium verfügbares Volumen von 10 - 20 cm$^3$, vorzugsweise ca. 15 cm$^3$ aus. Die gaspermeable Wand ist vorzugsweise in der Kulturkammereinheit lösbar, insbesondere austauschbar angeordnet. Dies ist vorteilhaft für die Reinigung und für die Verwendung unterschiedlicher gaspermeabler Wände.

Das erfindungsgemäße Verfahren zur Perifusionskultivierung von insbesondere menschlichen und tierischen Zellen, in einer mit Kulturmedium gefüllten Kulturkammer, bei dem das Kulturmedium innerhalb der Kulturkammer in Verbindung mit einem Gasgemisch steht und ein Gasaustausch zwischen dem Gasgemisch und dem Kulturmedium innerhalb der Kulturkammer durch eine das Gas vom Kulturmedium trennende gaspermeable Wand hindurch vorgenommen wird, ist dadurch gekennzeichnet, daß die Zellen in der Kulturkammer kontinuierlich mit Kulturmedium umspült werden, das vor dem Eintritt in die Kulturkammer mit einem Gasgemisch gesättigt wird, das Kulturmedium außerhalb der Kulturkammer kühler gehalten wird als in derselben und beim Eintritt in den dafür vorgesehenen Raum der Kulturkammer angewärmt und von abgeschiedenen Gasen befreit wird und daß das Kulturmedium den dafür vorgesehenen Raum der Kammer laminar im wesentlichen gleichmäßig über den gesamten Querschnitt des Raumes zwischen der gaspermeablen Wand und der Zellkultur bzw. deren Träger durchströmt.

Die Strömungsrichtung von Gas und Kulturmedium kann beliebig vorgesehen sein, wobei zumindest während der Zeit der Befüllung des Raumes für das Kulturmedium eine Strömung von unten nach oben bevorzugt ist. Der für das Kulturmedium und in der Kulturkammer vorhandene Raum ist im Betrieb des Perifusionsgerätes vorzugsweise frei von einer gasförmigen Phase, abgesehen von etwaigen zusätzlich vorgesehenen Gasabscheidern außerhalb des Bereiches des Trägers. Die kultivierten Zellen können somit, unabhängig von der Lage der Kulturkammer, stets mit Flüssigkeit bedeckt gehalten werden. Für mehrere Kulturkammereinheiten können gemeinsame Reservoire für das Kulturmedium vorgesehen sein. Bevorzugt sind jedoch gesonderte Reservoire für jede Kulturkammereinheit. Dadurch, daß die Kulturkammereinheiten thermisiert werden, kann das Kulturmedium außerhalb der Kulturkammer, beispielsweise im Reservoir, mit Vorteil kühler gehalten werden als in der Kulturkammereinheit, was aus Gründen der Sterilität bevorzugt ist. Beim Erwärmen des Kulturmediums etwa austretende Gase werden dann vor Eintritt des Kulturmediums in die eigentliche Kammer abgeschieden. Ein Austreten von Gasen vor der Kammereinheit kann dadurch auf einem Minimum gehalten werden, daß der Oxygenator und die Kammer auf demselben Temperaturniveau gehalten werden.

Die Zellen werden auf der bzw. den Seiten des Trägers kultiviert, die der gaspermeablen Wand zugewandt sind, wodurch ein guter Gasaustausch gewährleistet ist. Kulturmedium und Gas werden vorzugsweise im Gegenstrom zueinander geführt. Die Strömungsgeschwindigkeit des Kulturmediums durch die Kammer hängt unter anderem von der Haftfähigkeit der Zellen am Träger ab. Bei einem Strömungsspalt von etwa einem Millimeter zwischen Träger und gaspermeabler Wand kann der Durchsatz des Kulturmediums durch die Kammer bei etwa 0,2 Kammervolumina pro Minute gehalten werden.

Weitere Merkmale der Erfindung ergeben sich aus der nachfolgenden Beschreibung von bevorzugten Ausführungsformen in Verbindung mit der Zeichnung und den Ansprüchen. Hierbei können die einzelnen Merkmale jeweils für sich allein oder zu mehreren in Kombination miteinander bei einzelnen Ausführungsformen verwirklicht sein.

In der Zeichnung zeigen:

Fig. 1 eine schematische perspektivische Ansicht einer bevorzugten Ausführungsform der Erfindung in Modulbauweise,

Fig. 2 eine perspektivische schematische Darstellung einer einzelnen Moduleinheit,

Fig. 3 einen Vertikal schnitt durch eine Moduleinheit entlang der Linie III-III nach den Fig. 5 und 6 in verkleinertem Maßstab,

Fig. 4 einen Schnitt durch die Ausführungsform nach Fig. 3 entlang der Linie IV-IV nach den Fig. 5 und 6 in verkleinertem Maßstab,

Fig. 5 einen Schnitt durch die Ausführungsform nach Fig. 3 entlang der Linie V-V und

Fig. 6 einen Schnitt durch die Ausführungsform nach Fig. 3 entlang der Linie VI-VI.

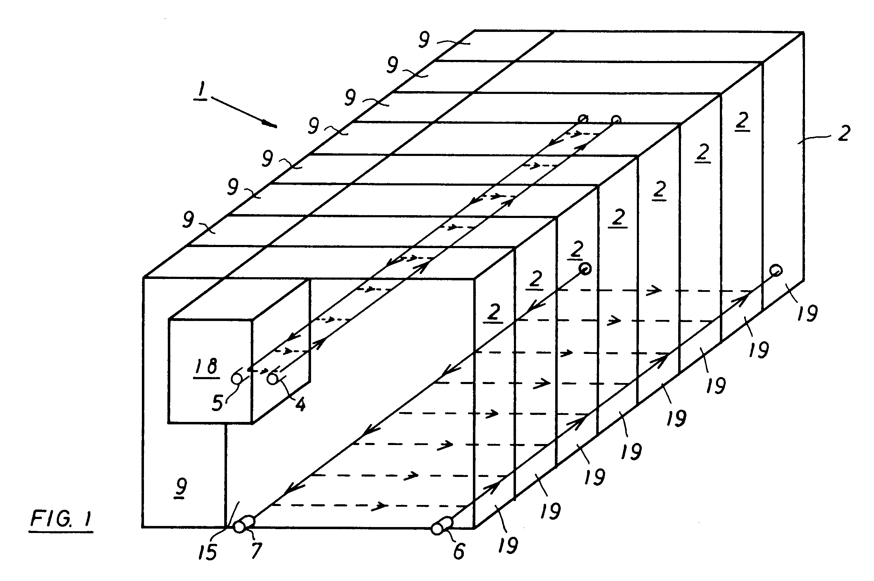

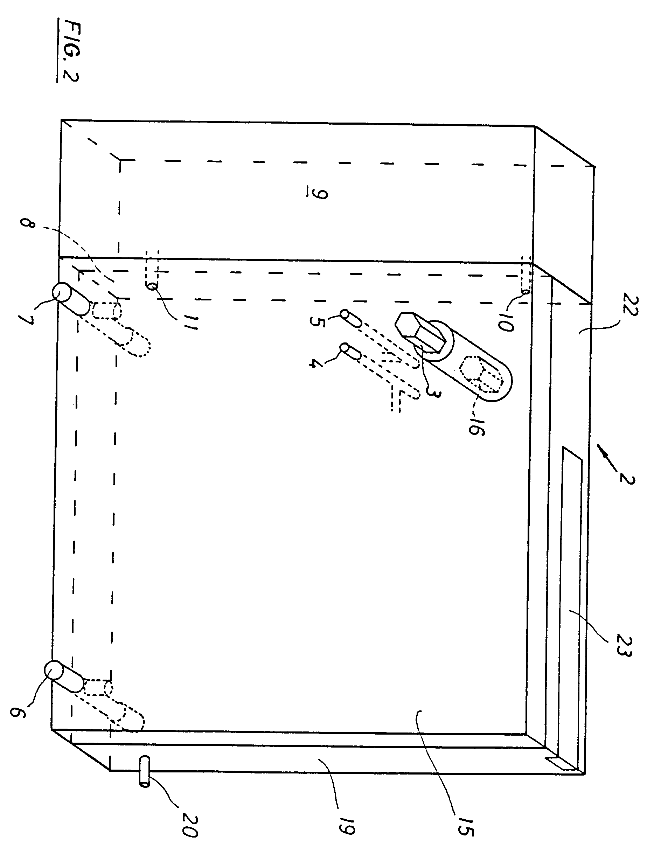

Bei der in der Zeichnung dargestellten Ausführungsform der Erfindung ist ein Perifusionsgerät 1 aus acht Moduleinheiten 2 zusammengesetzt, die quaderförmig ausgebildet sind und entlang großer Flächenseiten über Steckverbindungen 3, 4, 5, 6 und 7 miteinander verbindbar sind (Fig. 1 und 2).

Die Moduleinheiten 2 stehen aufrecht und liegen mit Flächenkontakt aneinander. Jede Moduleinheit 2 ist an einer Schmalseite 8 mit einem getrennten Reservoir 9 für Kulturmedium über Steckverbindungen 10 und 11 verbunden, die als Verbindungsleitungen für das Kulturmedium dienen. In jeder Moduleinheit 2 befindet sich eine Rollen- bzw. Schlauchpumpe 12, mittels derer Kulturmedium aus dem Reservoir 9 angesaugt und über einen Oxygenator 13 in eine Kulturkammereinheit 14 gedrückt werden kann. Der Antrieb der Schlauchpumpe 12 erfolgt mechanisch über Wellen mit 6-kant-Steckzapfen 3, die aus einer Flächenseite 15 jeder Moduleinheit herausragen und in eine entsprechende Ausnehmung 16 der gegenüberliegenden Flächenseite 17 der benachbarten Moduleinheit 2 eingreifen. In ähnlicher Weise ragen auf derselben Seite 15 Steckzapfen 5 und 6 von Gasleitungen und Steckzapfen 7 und 8 von Flüssigkeitsleitungen heraus und sind entsprechende Aufnahmen in der gegenüberliegenden Seite 17 vorgesehen. Die Steckverbindungen für Gase und Flüssigkeiten sind mit nicht dargestellten Steckventilen versehen, die nur in eingestecktem Zustand offen sind. Die Strömungsrichtung der Fluide ist in Fig. 1 schematisch durch Pfeile angezeigt. Ein Antriebsmotor 18 sitzt auf dem Steckzapfen 3 an der Flächenseite 15 der ersten Moduleinheit 2 und treibt sämtliche Schlauchpumpen 12 synchron an.

An der vom Reservoir 9 abweisenden Schmalseite 19 der Moduleinheit 2 ist unten weiterhin ein Auslaß 20 für Kulturmedium vorgesehen, der dann benützt wird, wenn anstelle einer Umwälzung von Kulturmedium durch die Moduleinheit 2 lediglich eine Durchströmung vorgesehen ist. Jede Moduleinheit 2 weist auf der vorderen Flächenseite 15 eine sich über diese gesamte Seite erstreckende Thermisiereinrichtung 21 auf, die über die Steckverbindungen 7 und 8 mit Thermisierflüssigkeit beschickbar ist. Die Thermisiereinrichtungen 21 sorgen auch in den benachbarten Moduleinheiten 2 für die Aufrechterhaltung der gewünschten Temperatur. Die hinterste Moduleinheit 2 kann an ihrer Rückseite 17 mit einer zusätzlichen Thermisiereinrichtung 21 versehen sein, so daß alle Moduleinheiten beidseitig an ihren großen Flächenseiten 15 und 17 thermisierbar sind.

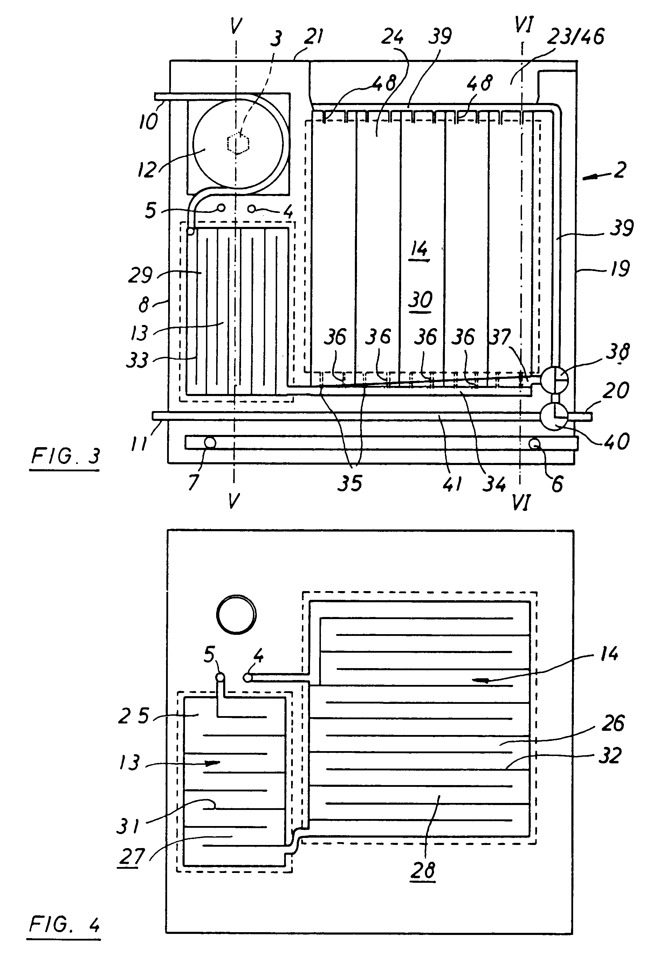

Aus Fig. 3 ist zu ersehen, daß sich in der Moduleinheit 2 die Pumpe 12, der Oxygenator 13 und die Kammereinheit 14 in einer Vertikalebene neben- bzw. übereinander befinden, wobei nicht nur der Oxygenator 13, sondern auch die Kulturkammereinheit 14 durch parallel zu den großen Seitenflächen 15 und 17 verlaufende gaspermeable Membranen 25 bzw. 26 jeweils in einen Raum 27 bzw. 28 für das Gas und einen Raum 29 bzw. 30 für das Kulturmedium getrennt sind. Die Membranen 25 und 26 bestehen aus flexiblem Silikonmaterial und sind auf der Gasseite mit labyrinthartig horizontal verlaufenden Stegen 31 bzw. 32 versteift. Dadurch ist das Volumen der Gas- bzw. Flüssigkeitsräume festgelegt. Gleichzeitig sorgen die Stege für eine gleichmäßige Bestreichung der Membran mit dem entlangströmenden Gas. Das Gas wird senkrecht zu den großen Flächenseiten 15, 17 durch die Steckverbindung 4 eingeführt, strömt im Gegenstrom zum Kulturmedium zuerst durch die Kulturkammereinheit 14 und dann durch den Oxygenator 13 und verläßt die Moduleinheit durch die Steckverbindung 5. Das Kulturmedium strömt von der Pumpe 12 zunächst in den neben der Kulturkammereinheit 14 und unterhalb der Pumpe 12 angeordneten Oxygenator 13, in dem es durch vertikal verlaufende Stege 33 umgelenkt wird. Das Kulturmedium fließt vom unteren Ende des Oxygenators 13 in das untere Ende der Kulturkammereinheit 14 entlang eines schräg nach oben verlaufenden Zuleitungskanales 34, der sich über die gesamte Breite der Kammereinheit 14 erstreckt. Der Zuleitungskanal 34 besitzt unterhalb seiner inneren Oberkante einzelne Öffnungen 35, durch die das Kulturmedium über kurze vertikale Leitungen 36 durch den Boden 37 des Flüssigkeitsraumes 30 in diesen eintritt. Etwaige Gasblasen, die sich vor Erreichen des Flüssigkeitsraumes vom Kulturmedium abgeschieden haben, sammeln sich im Zuleitungskanal 34 oberhalb der Eintrittsöffnungen 35, so daß der Zuleitungskanal als Gasabscheider dient. Am Ende 37 des Zuleitungskanales 34 ist ein Dreiweg-Ventil 38 angeordnet, das mit der vom oberen Ende des Flüssigkeitsraumes 30 der Kammereinheit kommenden Rücklaufleitung 39 für das Kulturmedium verbunden ist und über ein zweites Dreiweg-Ventil 40 entweder zum direkten Auslaß 20 oder über eine Umwälzrückleitung 41 zur Steckverbindung 11 und damit zum Reservoir 9 zurückführt. Durch das Dreiweg-Ventil 38 kann die Luft aus dem Gasabscheider abgelassen werden. Das Kulturmedium strömt im Zuleitungskanal 34 und in der Rücklaufleitung 39 in derselben Richtung. Dadurch wird erreicht, daß das Kulturmedium unabhängig davon, ob es bereits am Anfang des Zuleitungskanals 34 in den Flüssigkeitsraum 30 oder erst am Ende des Zuleitungskanals 34 in diesen fließt, bis zur Rücklaufleitung 39 stets die gleiche Weglänge durchströmt.

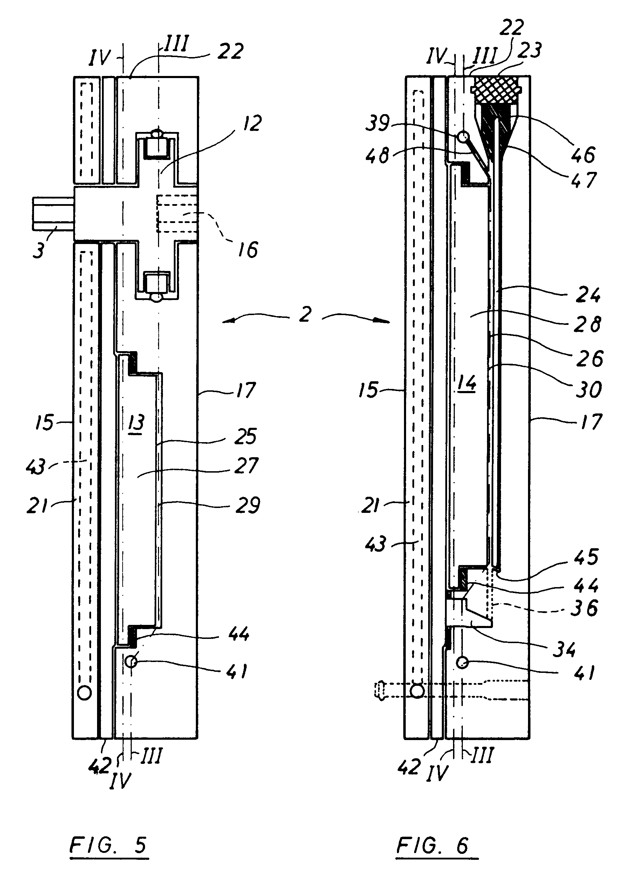

Die Moduleinheit 2 ist, wie aus den Fig. 5 und 6 zu ersehen ist, im Querschnitt mehrschichtig aufgebaut. An die Rückwand 17 schließt sich die Kammereinheit 14 bzw. der Oxygenator 13 an, auf denen eine Abdeckplatte 42 angeordnet ist, die lediglich von der Pumpe 12 bzw. deren Antriebszapfen 3 durchbrochen ist. Über der Abdeckplatte 42 liegt die Thermisierungseinrichtung 21, die mit Kanälen 43 für die Thermisierungsflüssigkeit durchzogen ist. Der Oxygenator 13 besitzt einen im Verhältnis zum Flüssigkeitsraum 29 großen Gasraum, der durch die flächige Membran 25 und eine Dichtung 44 vom parallel verlaufenden Flüssigkeitsraum getrennt ist. Bei der Kulturkammereinheit 14 liegen die Verhältnisse entsprechend, doch befindet sich dort im Flüssigkeitsraum 30 die Trägerplatte 24 in einem lichten Abstand von ca. 1 mm von der Membran 26. Im Zwischenraum werden die vom Kulturmedium laminar beströmten Zellen einschichtig auf der Trägerplatte 24 haftend kultiviert. Der Flüssigkeitsraum 30 und die Trägerplatte 24 sind in fünf vertikale Teilkammern bzw. Streifen für die Durchführung von parallelen Kultivierungen unterteilt. Die Justierung der Trägerplatte am unteren Ende erfolgt durch eine Abschrägung 45, durch die die Trägerplatte an der Kammerrückwand gehalten wird. Am oberen Ende ist die Trägerplatte mit einem Dichtprofil 46 versehen, das durch den als Schieber ausgebildeten Deckel 23 in eine im Schnitt trichterförmige Einführschräge 47 für die Trägerplatte 24 eingedrückt wird. Kleine Stichkanäle 48 zweigen unterhalb der Einführschräge 47 aber oberhalb der Membran 26 aus jeder Teilkammer zur Rücklaufleitung 39 ab, so daß der Raum unterhalb des Dichtprofiles wiederum als Gasabscheider dienen und beim Entnehmen der Trägerplatte entlüftet werden kann. Der für das Kulturmedium vorgesehene Raum ist somit im Betrieb stets vollständig mit dem Kulturmedium gefüllt.

Beim Betreiben des Perifusionsgerätes 1 können ganze Moduleinheiten 2 oder einzelne Trägerplatten 24, z.B. zur Kontrolle des Zellwachstums oder zur Probe-Entnahme herausgenommen werden, ohne daß dadurch die anderen Moduleinheiten beeinträchtigt werden. Es kann in den Moduleinheiten unter vergleichbaren Bedingungen gearbeitet werden, wodurch die Aussagekraft der Kultivierungsergebnisse wesentlich verbessert wird. Dadurch, daß die Moduleinheiten blockartig ausgebildet sind, haben sie Standflächen, was die Handhabung wesentlich erleichtert. Im übrigen können die einzelnen Formteile der Moduleinheiten aus Kunststoff, beispielsweise durch Spritzguß, geformt sein und abdichtend miteinander verbunden werden, was eine einfache Herstellung und gute Reinigung ermöglicht.

**Patentansprüche**

1. Perifusionsgerät (1) für die Züchtung lebender Zellen mit mindestens einer Kulturkammereinheit (14) zur Aufnahme von flüssigem Kulturmedium und Gas, mindestens einem Oxygenator (13) für das Kulturmedium, mindestens einer Flüssigkeitspumpe (12) für das Kulturmedium und mindestens einem Reservoir (9) für das Kulturmedium, dadurch gekennzeichnet, daß die Kulturkammereinheit (14) in einen Flüssigkeitsraum (30) und einen Gasraum (28) unterteilt ist und hierzu der Raum (30) für das Kulturmedium in der Kulturkammereinheit (14) durch mindestens eine gaspermeable Wand (26) von dem Raum (28) für das Gas abgetrennt ist, in der Kulturkammereinheit (14) im Raum (30) für das Kulturmedium mindestens ein plattenförmiger Träger (24) für eine Monolayer-Kultur im wesentlichen parallel zur gaspermeablen Wand (26) angeordnet ist, die Kulturkammereinheit (14) und die gaspermeable Wand (26) im wesentlichen vertikal angeordnet sind, der Raum (30) für das Kulturmedium für eine vollständige Füllung mit dem Kulturmedium im Betrieb ausgebildet ist, der Flüssigkeitsraum (30) der Kulturkammereinheit (14) laminar und im wesentlichen gleichmäßig über den gesamten Querschnitt des Raumes zwischen der gaspermeablen Wand (26) und dem Träger (24) mit Kulturmedium durchströmbar ist und an der Eintrittsstelle für das Kulturmedium im unteren Bereich der Kulturkammereinheit (14) ein Gasabscheider (34) vorgesehen ist.

2. Perifusionsgerät nach Anspruch 1, dadurch gekennzeichnet, daß die Kulturkammereinheit (14) platten- bzw. tafelförmig ausgebildet ist und die gaspermeable Wand (26) im wesentlichen parallel zu einer großen Flächenseite (15, 17) der Kulturkammereinheit (14) als Trennwand in dieser angeordnet ist.

3. Perifusionsgerät nach Anspruch 1 oder 2, dadurch gekennzeichnet, daß mehrere, vorzugsweise mindestens fünf Kulturkammereinheiten (14) jeweils in getrennten Moduleinheiten (2) vorgesehen sind, die von anderen Moduleinheiten (2) und gegebenenfalls vom Vorratsbehälter (9) für das Kulturmedium abnehmbar und insbesondere über lösbare Steckverbindungen mit diesen verbindbar sind.

4. Perifusionsgerät nach Anspruch 3, dadurch gekennzeichnet, daß die einzelnen abnehmbaren Moduleinheiten (2) mit einem eigenen Oxygenator (13) und vorzugsweise einer eigenen Umwälzpumpe (12) ausgerüstet sind.

5. Perifusionsgerät nach Anspruch 3 oder 4, dadurch gekennzeichnet, daß die vorzugsweise flächig bis plattenartig ausgebildeten Moduleinheiten (2) dicht nebeneinander angeordnet sind und insbesondere durch gegenseitige Berührung thermisch miteinander koppelbar sind.

**6.** Perifusionsgerät nach einem der vorhergehenden Ansprüche, dadurch gekennzeichnet, daß der im Raum (30) für das Kulturmedium vorgesehene Träger (24) für die Monolayer-Kultur herausnehmbar ist und insbesondere in einzelne parallele Streifen unterteilt ist.

**7.** Perifusionsgerät nach einem der vorhergehenden Ansprüche, dadurch gekennzeichnet, daß die gaspermeable Wand (26) in der Kulturkammereinheit (14) lösbar, insbesondere austauschbar, angeordnet ist.

**8.** Verfahren zur Perifusions-Kultivierung von insbesondere menschlichen und tierischen Zellen in einer mit Kulturmedium gefüllten Kulturkammer, bei dem das Kulturmedium innerhalb der Kulturkammer in Verbindung mit einem Gasgemisch steht und ein Gasaustausch zwischen dem Gasgemisch und dem Kulturmedium innerhalb der Kulturkammer durch eine das Gas vom Kulturmedium trennende gaspermeable Wand hindurch vorgenommen wird, dadurch gekennzeichnet, daß die Zellen in der Kulturkammer kontinuierlich mit Kulturmedium umspült werden, das vor dem Eintritt in die Kulturkammer mit einem Gasgemisch gesättigt wird, das Kulturmedium außerhalb der Kulturkammer kühler gehalten wird als in derselben und beim Eintritt in den dafür vorgesehenen Raum der Kulturkammer angewärmt und von abgeschiedenen Gasen befreit wird und daß das Kulturmedium den dafür vorgesehenen Raum der Kammer laminar im wesentlichen gleichmäßig über den gesamten Querschnitt des Raumes zwischen der gaspermeablen Wand und der Zellkultur bzw. deren Träger durchströmt.

**9.** Verfahren nach Anspruch 8, dadurch gekennzeichnet, daß das den Gasraum der Kammer durchströmende Gas im Gegenstrom zum Kulturmedium geführt wird.

## Claims

**1.** Perifusion apparatus (1) for culturing living cells with at least one culture chamber unit (14) for receiving liquid culture medium and gas, at least one oxygenator (13) for the culture medium, at least one hydraulic pump (12) for the culture medium and at least one reservoir (9) for the culture medium, characterized in that the culture chamber unit (14) is subdivided into a liquid area (30) and a gas area (28) and for this purpose the area (30) for the culture medium in the culture chamber unit (14) is separated by at least one gas-permeable wall (26) from the gas area (28), in the culture media area (30) within the culture chamber unit (14) is provided at least one plate-like carrier (24) for a monolayer culture substantially parallel to the gas-permeable wall (26), the culture chamber unit (14) and the gas-permeable wall (26) are positioned substantially vertically, the culture medium area (30) is constructed for a complete filling with the culture medium in operation, the culture medium can flow in laminar, substantially uniform manner through the liquid area (30) of the culture chamber unit (14) over the entire cross-section of the area between the gas-permeable wall (26) and the carrier (24) and a gas separator (34) is provided at the intake point for the culture medium in the lower area of the culture chamber unit (14).

**2.** Perifusion apparatus according to claim 1, characterized in that the culture chamber unit (14) is constructed in plate-like or tabular manner and the gas-permeable wall (26) is substantially parallel to a large surface side (15, 17) of the culture chamber unit (14) as a partition therein.

**3.** Perifusion apparatus according to claims 1 or 2, characterized in that several, preferably at least five culture chamber units (14) are provided in separate module units (2), which are removable from other module units (2) and optionally from the culture medium reservoir (9) and are connectable thereto in particular by means of detachable plug connections.

**4.** Perifusion apparatus according to claim 3, characterized in that the individual removable module units (2) are equipped with their own oxygenator (13) and preferably their own circulating pump (12).

**5.** Perifusion apparatus according to claims 3 or 4, characterized in that the preferably flat or plate-like module units (2) are closely juxtaposed and can in particular be thermally coupled together by reciprocal contact.

**6.** Perifusion apparatus according to one of the preceding claims, characterized in that the carrier (24) for the monolayer culture provided in the area (30) for the culture medium is removable and in particular subdivided into individual parallel strips.

**7.** Perifusion apparatus according to one of the preceding claims, characterized in that the gas-permeable wall (26) is detachably and in particular replaceably placed in the culture chamber unit (14).

8. Process for the perifusion culturing of in particular human and animal cells in a culture chamber filled with culture medium, in which the culture medium is connected to a gaseous mature within the culture chamber and a gas exchange between the gaseous mixture and the culture medium within the culture chamber is carried cut through a gas-permeable wall separating the gas from the culture medium, characterized in that culture medium continuously flows round the cells in the culture chamber, having been saturated with a gaseous mixture prior to entering the culture chamber, the culture medium is kept cooler outside the culture chamber than in the latter and on entering the culture chamber area provided for this purpose it is initially heated and freed from separated gases and that the culture medium flows in laminar manner through the provided chamber area substantially uniformly over the entire cross-section of the area between the gas-permeable wall and the cell culture or its carrier.

9. Process according to claim 8, characterized in that the gas flowing through the gas area of the chamber is in counterflow to the culture medium.

**Revendications**

1. Dispositif de périfusion (1) pour la culture de cellules vivantes comportant au moins une chambre de culture (14) pour recevoir le milieu de culture liquide et le gaz, au moins un dispositif d'oxygénation (13) pour le milieu de culture, au moins une pompe à liquide (12) pour le milieu de culture et au moins un réservoir (9) pour le milieu de culture, caractérisé en ce que la chambre de culture (14) est subdivisée en un compartiment pour liquide (30) et un compartiment pour gaz (28) et que, par ailleurs, le compartiment (30) pour le milieu de culture dans la chambre de culture (14) est séparé par, au moins, une paroi perméable au gaz (26) du compartiment (28) pour le gaz, en ce que la chambre de culture (14) comporte dans le compartiment (30) pour le milieu de culture au moins un support (24) en forme de plaque pour une culture monocouche essentiellement parallèle à la paroi perméable au gaz (26), en ce que la chambre de culture (14) et la paroi perméable au gaz (26) sont disposées essentiellement à la verticale, en ce que le compartiment (30) pour le milieu de culture est conçu pour un remplissage complet avec le milieu de culture en service, en ce que le compartiment pour liquide (30) de la chambre de culture (14) peut être pénétré par le milieu de culture de manière laminaire et essentiellement régulière sur toute la section du compartiment entre la paroi perméable au gaz (26) et le support (24) et en ce qu'un séparateur de gaz (34) est prévu à l'entrée du milieu de culture dans la région inférieure de la chambre de culture (14).

2. Dispositif de périfusion, selon la revendication 1, caractérisé en ce que la chambre de culture (14) est conçue en forme de plaque ou de tablette et en ce que la paroi perméable au gaz (26) est essentiellement parallèle à un grand côté de la surface (15, 17) de la chambre de culture (14) comme paroi de séparation de celle-ci.

3. Dispositif de périfusion, selon la revendication 1 ou 2, caractérisé en ce que plusieurs chambres de culture (14), de préférence au moins cinq, sont prévues, respectivement en unités modulaires séparées (2) qui peuvent être extraites des autres unités modulaires (2) et, éventuellement, du réservoir (9) pour le milieu de culture et peuvent en particulier être fixées à celles-ci en particulier par des fixations amovibles à enficher.

4. Dispositif de périfusion, selon la revendication 3, caractérisé en ce que les différentes unités modulaires (2) amovibles sont équipées d'un dispositif d'oxygénation propre (13) et de préférence d'une pompe de circulation propre (12).

5. Dispositif de périfusion, selon la revendication 3 ou 4, caractérisé en ce que les unités modulaires (2) de conception de préférence plane jusqu'à une conception en forme de plaque sont disposés l'une à côté de l'autre et peuvent en particulier être couplées thermiquement l'une à l'autre par contact mutuel.

6. Dispositif de périfusion selon une des revendications précédentes caractérisé en ce que le support (24) prévu dans le compartiment (30) pour le milieu de culture peut être extrait pour la culture monocouche et est en particulier subdivisé en bandes parallèles individuelles.

7. Dispositif de périfusion selon une des revendications précédentes caractérisé en ce que la paroi perméable au gaz (26) est disposée dans la chambre de culture (14) de manière amovible, en particulier interchangeable.

8. Procédé de culture par périfusion de cellules, particulièrement humaines et animales, dans une chambre de culture remplie de milieu de culture, selon lequel le milieu de culture est en contact avec un mélange gazeux dans la chambre de culture et qu'il se produit un échange gazeux entre le mélange gazeux et le milieu de culture dans la chambre de culture par une paroi perméable au gaz séparant le gaz du milieu de culture, caractérisé en ce que les cellules sont rincées en continu dans la chambre de culture avec le milieu de culture qui est saturé avec un mélange gazeux avant l'entrée dans la chambre de culture, que le milieu de culture est maintenu à l'extérieur de la chambre de culture à une température inférieure à celle à l'intérieur de ladite chambre de culture et est chauffé lors de son entrée dans le compartiment de la chambre de culture prévu à cet effet et libéré des gaz séparés et que le milieu de culture parcourt le compartiment prévu dans la chambre de manière laminaire, essentiellement régulière par toute la section du compartiment entre la paroi perméable au gaz et la culture cellulaire ou son support.

9. Procédé, selon la revendication 8, caractérisé en ce que le gaz pénétrant dans le compartiment pour gaz de la chambre est amené à contre-courant au milieu de culture.

FIG. 1

FIG. 2

EP 0 230 223 B1

FIG. 3

FIG. 4

FIG. 5

FIG. 6